# EUROPEAN PATENT APPLICATION

(11) **EP 1 177 806 A1**
(43) Date of publication of application: **06.02.2002**
(21) Application number: 00306645.3
(22) Date of filing: 04.08.2000
(51) Int. Cl.: A61M 15/00

(54) **Dry powder inhaler**

(71) Applicant: THE TECHNOLOGY PARTNERSHIP PUBLIC LIMITED COMPANY, Melbourn Royston Hertfordshire SG8 6EE (GB)
(72) Inventor: McDerment, Iain Grierson, Royston, Hertfordshire SG8 6DB (GB); Bowman, Nicholas John, Royston, Hertfordshire SG8 5QE (GB); Angel, Clive Graham, Royston, Hertfordshire SG8 6ET (GB)
(74) Representative: Brunner, Michael John

(57) **Abstract**

A dry powder inhaler device 1 has a flat housing with a chamber 3 for inhalant medicament and an inhalant outlet 9 including a mouthpiece 10. An air inlet 19 is provided, together with a disc like rotor 13 having an inhalant recess 18 arranged at its edge, the rotor being disposed within a recess 12 in the housing sized to closely fit the disc. The inhalant recess 18 is movable around the axis of the rotor from a first position in which it is in communication with the chamber 3 to a second, dispensing position in which both the air inlet 8 and the outlet 9 are in communication with the inhalant recess. Thus, in use, a dose of powdered inhalant is removed from the chamber 3 in the recess 18 as the rotor is rotated 13 and is provided to a position at which it can be inhaled by a user by being drawn out of the inhalant recess entrained in a flow of air through the inlet 19, the inhalant recess 18, and the inhalant outlet 9 and mouthpiece 10.

## Description

The present invention relates to inhalers and, more particularly to inhalers which use a dry powder as the inhalant and which provide multiple metered inhalant doses.

It is known to provide inhalers with pre-metered doses in a convenient package size such as to allow users to carry an inhaler in a handbag, pocket or the like. However, conventional inhaler devices, which may be re-usable and, for example, utilise a strip of frangible receptacles each containing a dose of the medicament to be inhaled, the strip being pulled through the inhaler in steps to allow each receptacle to be fractured in use to dispense the medicament, are bulky and, more importantly from a cost and reliability point of view, contain numerous parts which have to be accurately assembled and work consistently over time.

Other multiple-dose inhalers also tend to be bulky.

There is therefore a need for an inhaler of this general type which is both simple to assemble and reliable in use, but which is also small.

According to the present invention there is provided a dry powder inhaler device including
a flat housing having a chamber for inhalant medicament and an inhalant outlet including a mouthpiece;
an air inlet; and,
a disc like rotor having an inhalant recess arranged at its edge, the rotor being disposed within a recess in the housing sized to closely fit the disc, and the inhalant recess being movable, around the axis of the rotor from a first position in which it is in communication with the chamber to a second, dispensing position in which both the air inlet and the outlet are in communication with the inhalant recess, whereby in use a dose of powdered inhalant is removed from the chamber in the recess as the rotor is rotated and is provided to a position at which it can be inhaled by a user by being drawn out of the inhalant recess entrained in a flow of air through the inlet, the inhalant recess, and the inhalant outlet and mouthpiece.

The mouthpiece preferably has a hinged cover and an arm supported on the rotor and the cover is supported on the arm for rotation about the axis of the rotor. The housing halves may be formed from a single moulded plastics blank folded around the rotor during assembly.

The housing can be approximately credit-card size in area and have a thickness of say 5 to 7mm to allow the inhaler to be easily carried in a pocket, wallet or purse.

Preferably, the air inlet is formed in the rotor. The inhaler may include a pair of air inlets and a pair of air outlets, a primary air inlet in the housing connecting directly with a primary air outlet and a secondary air inlet in the rotor connecting with a secondary air outlet via the recess when it is at the dispensing position, and the secondary air outlet connecting into the primary air outlet.

The use of dry powder requires that ambient moisture be prevented from reaching the pre-metered doses and this may be achieved either by arranging for a sealing fit between the rotor and the walls of the chamber in which the rotor is disposed and by suitable selection of the materials of both the rotor and the housing, or else by providing seals around and/or over the inhalant recess.

Preferably, the housing has a transparent window at the dispensing position of the inhalant recess allowing the user to confirm the presence of the inhalant dose before using the device and the complete inhalation of the dose after use. This may be provided by manufacturing the housing from a transparent material and masking the surface of the housing (eg by printing) and leaving an unmasked area at the dispensing position of the inhalant recess.

Similarly, it is preferable to provide a window over the chamber so that the user can see the amount of medicament remaining. Again, this may be provided by a transparent housing suitably masked by printing except over the chamber.

It will be appreciated that the design of the device according to the invention may include a very small number of parts, significantly reducing material and assembling costs relative to prior inhalers.

A further advantage of this design is the provision of a large flat printing area on the faces of the housing, allowing plenty of room for the printing of instructions, product information and the like.

An example of an inhaler device according to the invention will now be described with reference to the accompanying drawings, in which:
Figure 1 is a side view;
Figure 2 is a first sectional view showing the inhaler in a first position;
Figure 3 is a second sectional view showing the inhaler in a second position;
Figure 4 is a plan view of a first housing part;

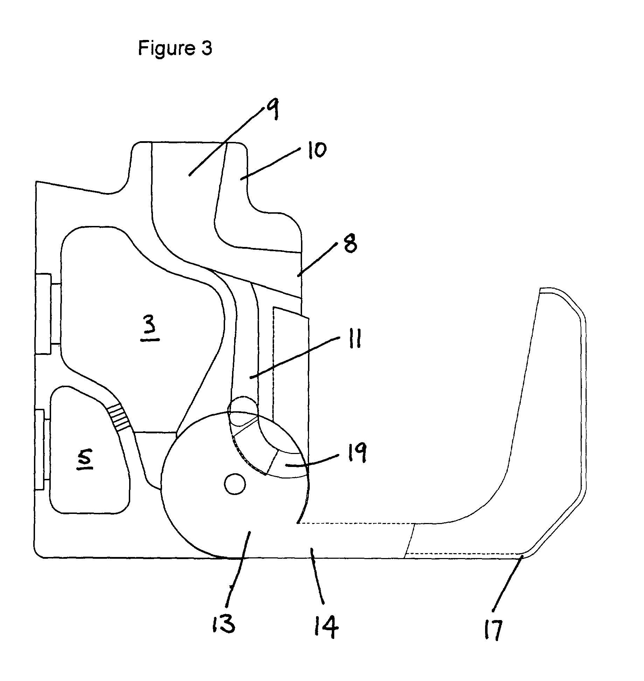

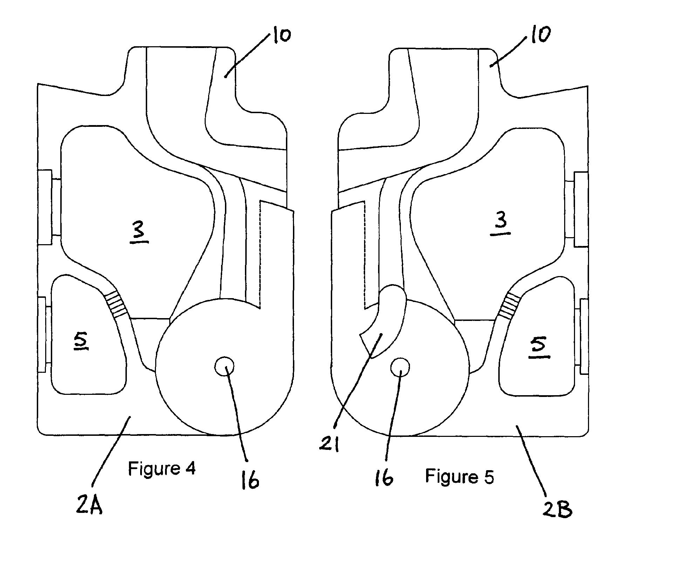

Figure 5 is a plan view of a second housing part;

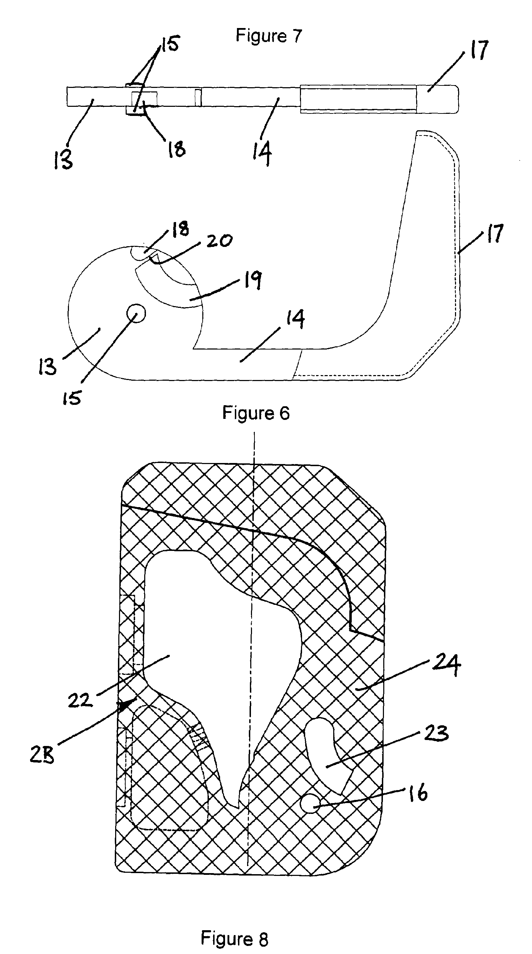

Figure 6 is a plan view of a cover/actuator component;
Figure 7 is a side view of the cover/actuator component; and,
Figure 8 is a face view of the inhaler showing areas of surface printing.

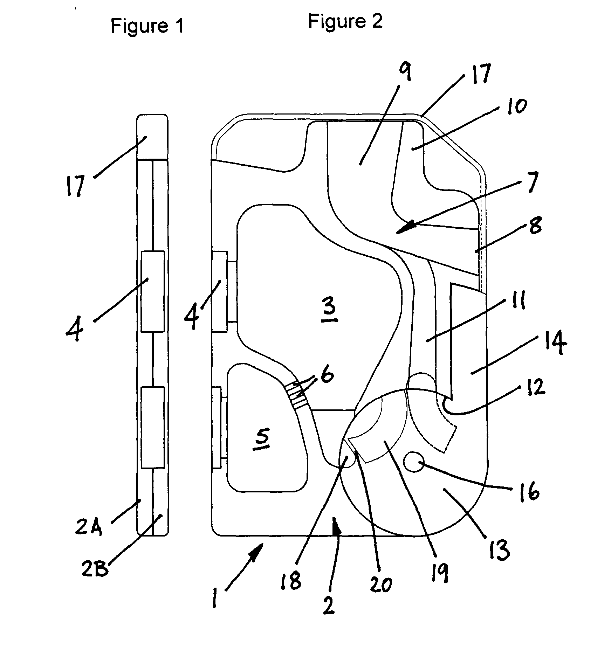

The inhaler 1 shown in the figures has a transparent polypropylene or other plastics housing 2 which, for the sake of convenience is shown, in figure 2, in two parts 2A,2B, but which may actually be formed from a single, hinged blank if desired. The housing is approximately credit-card size for convenience and has, formed between the two halves, a first, hopper-shaped chamber 3 which, in use, contains a powdered inhalant medicament (not shown) provided to the chamber through a filling hole 4. Preferably, sufficient inhalant is provided for 100 doses. A second chamber 5 in use contains a desicant which may be in the powdered form or in the form of a pre-shaped (eg. moulded) block (not shown), and communicates with the inhalant chamber 3 through a series of small passages 6 to prevent the inhalant from absorbing moisture and forming aggregations of powder.

A primary airflow channel 7 is formed between the two housing halves 2A,2B, having an inlet 8 at one side of the housing and an outlet 9 which extends though a projecting mouthpiece 10. A secondary airflow channel 11 connects to the primary channel and extends from the edge of a part-circular recess 12 formed at one corner of the housing 2. In the recess 12 is positioned a part circular, disc like polypropylene rotor 13 which is formed on the end of an arm 14 and which is rotatable in the housing around pivot protrusions 15 at the centre of the rotor 13 and which extend into pivot holes 16 in the housing halves. The arm 14 supports a cover 17 which closes the mouthpiece 10 (see figure 2) in one position and which opens the mouthpiece (see figure 3) in another position. In the closed position, the cover 17 also closes the primary airflow inlet 8 and in the open position it opens the primary airflow inlet 8.

The disc like rotor 13 has an inhalant dose recess or cup 18 (sized to contain the right amount of inhalant powder for a single dose) disposed in its edge and positioned so that it is normally (ie. when the cover is closed) communicating with the bottom of the chamber 3 and is filled with medicament powder from the chamber 3 when the inhaler is held upright with the mouthpiece uppermost. A secondary air inlet 19 is formed in the rotor 13 separated from the inhalant recess 18 by a narrow wall 20 and is normally closed from the outside (see figure 2). To provide a dose of inhalant, the cover 17 is opened (to the position shown in figure 3) and this causes the rotor 13 to rotate so that inhalant in the recess or cup 18 is provided to the end of the secondary airflow channel 11and the inlet 21 is opened to the outside. A shallow channel 21 is formed in the housing half 2B so as to provide a connection from the secondary air inlet 19 to the secondary airflow channel 11, the wall 20 acting to form a venturi. This ensures a pressure drop across the wall 20, in turn ensuring satisfactory entrainment of inhalant in the air flowing through the secondary airflow channel 11 from the secondary inlet 19 when the user inhalers via the mouthpiece 10. The connection of the secondary airflow channel 11 into the primary airflow channel 7 results in a pressure drop at the junction of the channels. The advantage of this is that powdered inhalant is fed gradually into the outlet airstream through the airflow channel 7. Depending on the specific use of the inhaler, the target users, the specific inhalant etc., the relative sizes of the channels may be arranged accordingly.

The exterior faces of the housing are printed as shown by the cross-hatching 24, in the case of the half 2B so as to reveal a first window 22 which is positioned over the chamber 3 and allows the user to see how much inhalant is left in it. A second window 23 is provided over the shallow channel 21 so that the user can see that a dose of medicament is in the recess 18 before inhaling and enables the user to check that all the medicament has been inhaled from the recess 8 at the dispensing position.

The window 22 may be over printed at appropriate positions with numerals indicating the approximate number of doses remaining in the chamber.

## Claims

1. A dry powder inhaler device including
a flat housing having a chamber for inhalant medicament and an inhalant outlet including a mouthpiece;
an air inlet; and,
a disc like rotor having an inhalant recess arranged at its edge, the rotor being disposed within a recess in the housing sized to closely fit the disc, and the inhalant recess being movable, around the axis of the rotor from a first position in which it is in communication with the chamber to a second, dispensing position in which both the air inlet and the outlet are in communication with the inhalant recess, whereby in use a dose of powdered inhalant is removed from the chamber in the recess as the rotor is rotated and is provided to a position at which it can be inhaled by a user by being drawn out of the inhalant recess entrained in a flow of air through the inlet, the inhalant recess, and the inhalant outlet and mouthpiece.

2. A dry powder inhaler device according to claim 1, wherein the housing is substantially credit-card size in area.

3. A dry powder inhaler device according to claim 1 or claim 2, wherein the housing has a thickness of between 5 and 7 mm.

4. A dry powder inhaler device according to any of claims 1 to 3, wherein the rotor and walls of the corresponding recess are a sealing fit.

5. A dry powder inhaler device according to any of claims 1 to 3, including seals around and/or over the recess.

6. A dry powder inhaler device according to any of claims 1 to 5, wherein the housing has a transparent window at the dispensing position of the recess, allowing the user to confirm the presence of the inhalant dose before using the device and check the complete inhalation of the dose after use.

7. A dry powder inhaler device according to any of claims 1 to 6, wherein the window is provided by forming the housing of a transparent material, masking the surface of the housing and leaving a portion of the housing unmasked at the window location.

8. A dry powder inhaler device according to any of claims 1 to 7, including a window over the chamber to allow the user to view the contents thereof.

9. A dry powder inhaler device according to any of claims 1 to 6, wherein the window is provided by forming the housing of a transparent material, masking the surface of the housing and leaving a portion of the housing unmasked at the window location.

10. A dry powder inhaler device according to any of claims 1 to 9, wherein the mouthpiece has a hinged cover.

11. A dry powder inhaler device according to claim 9, further including an arm supported on the rotor and wherein the cover is supported on the arm for rotation about the axis of the rotor.

12. A dry powder inhaler device according to any of claims 1 to 11, wherein the housing is formed from a single moulded plastics blank folded around the rotor during assembly.

13. A dry powder inhaler device according to any of claims 1 to 12, wherein the inlet is formed in the rotor.

14. A dry powder inhaler device according to any of claims 1 to 12, including a pair of air inlets and a pair of air outlets, a primary air inlet in the housing connecting directly with a primary air outlet and a secondary air inlet in the rotor connecting with a secondary air outlet via the recess when it is at the dispensing position, and the secondary air outlet connecting into the primary air outlet.

15. A dry powder inhaler device according to any of claims 1 to 14, further including a chamber for desicant arranged in communication with the inhalant chamber.
